(19) Europäisches Patentamt
European Patent Office
Office européen des brevets

(11) Veröffentlichungsnummer: **0 013 443**
**B1**

(12) **EUROPÄISCHE PATENTSCHRIFT**

(45) Veröffentlichungstag der Patentschrift: **31.03.82**

(51) Int. Cl.³: **C 07 D 211/46,** C 08 K 5/34

(21) Anmeldenummer: **79200441.8**

(22) Anmeldetag: **28.06.79**

(60) **Veröffentlichungsnummer oder, falls nicht vorhanden, Anmeldenummer der früheren Anmeldung nach** Art. 76 EPÜ: **7910214.5**

(54) **2,2,6,6-Tetramethylpiperidin-Derivate, Verfahren zu ihrer Herstellung und mit ihrer Hilfe stabilisierte synthetische Polymere.**

(30) Priorität: **03.07.78 GB 2865378**

(43) Veröffentlichungstag der Anmeldung: **23.07.80 Patentblatt 80/15**

(45) Bekanntmachung des Hinweises auf die Patenterteilung: **31.03.82 Patentblatt 82/13**

(84) Benannte Vertragsstaaten: **DE FR GB IT**

(56) Entgegenhaltungen:
**DE-A-2 258 752**
**DE-A1-2 656 769**
**GB-A-1 376 438**

(73) Patentinhaber: **CIBA-GEIGY AG, Patentabteilung Postfach, CH-4002 Basel (CH)**
Patentinhaber: **SANKYO COMPANY LIMITED, No. 1-6, 3-chome Nihonbashi Honcho Chuo-ku, Tokyo (JP)**

(72) Erfinder: **Rody, Jean, Dr., Rütiring 82, CH-4125 Riehen (CH)**

(74) Vertreter: **Stamm, Otto et al, Patentabteilung der CIBA-GEIGY AG Postfach, CH-4002 Basel (CH)**

**EP 0 013 443 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

2,2,6,6-Tetramethylpiperidin-Derivate,
Verfahren zu ihrer Herstellung und mit ihrer Hilfe stabilisierte synthetische Polymere

Die vorliegende Erfindung betrifft neue 2,2,6,6-Tetramethylpiperidin-Derivate und mit deren Hilfe stabilisierte synthetische Polymere.

In der JP-A-48-65 182 sind 2,2,6,6-Tetramethylpiperidin-Derivate der Formeln

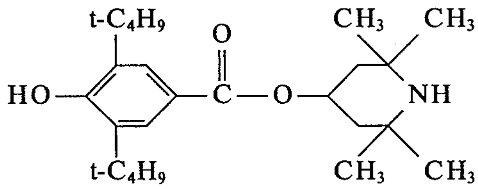

und

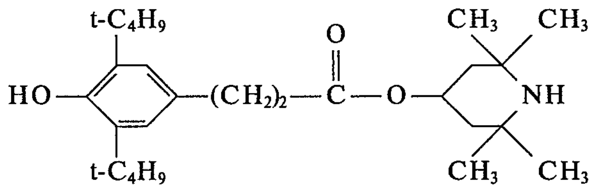

welche 3,5-Di-tert.butyl-4-hydroxyphenyl-gruppen enthalten, als Stabilisatoren für synthetische Polymere beschrieben worden. In der JP-A-48-65 180 sind die N-methyl-Derivate der oben erwähnten Verbindungen offenbart.

Es wurde nun gefunden, daß 2,2,6,6-Tetramethylpiperidin-Derivate, in welchen eine 3,5-Di-tert.butylphenylgruppe auch in 1-Stellung gebunden ist, hohe Lichtschutzwirkung und gleichzeitig hitzestabilisierende Wirkung sowie Verarbeitungsstabilisation entfalten. Die Verbindungen sind schwer flüchtig und besser verträglich mit dem Polymeren sowie extraktions-resistenter.

Die neuen Verbindungen entsprechen der Formel I

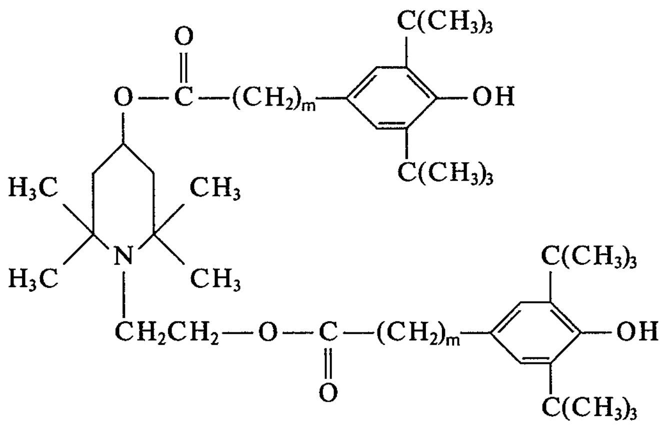

(I)

worin m 0, 1 oder 2 ist.

m ist vorzugsweise 2. Die erfindungsgemäßen Verbindungen haben die folgenden Strukturen:

1)

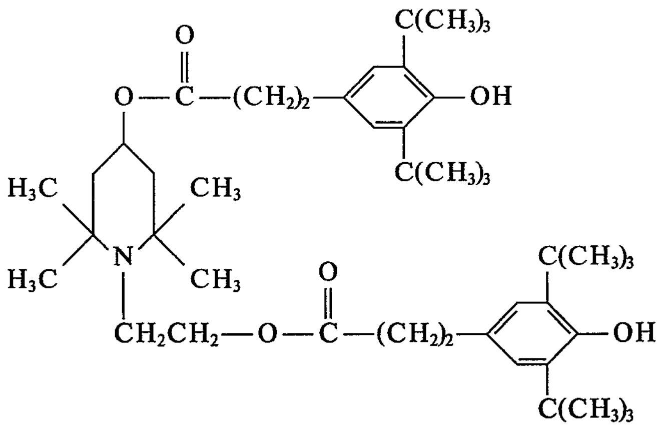

2)

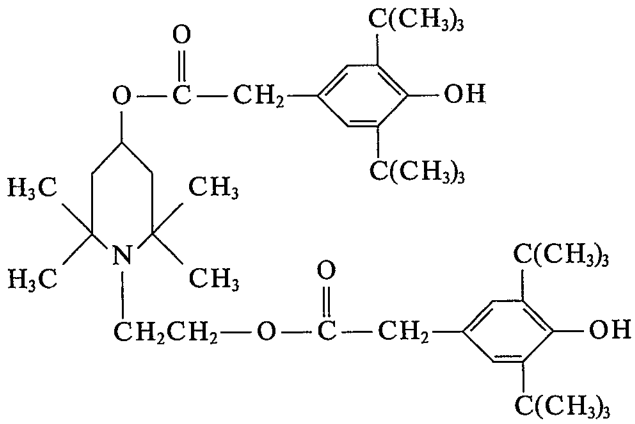

3)

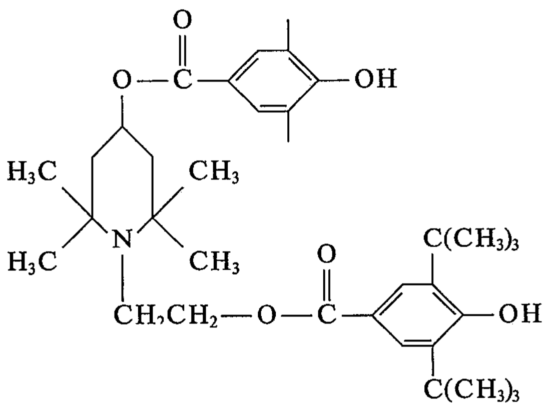

Die erfindungsgemäßen 2,2,6,6-Tetramethylpiperidin-Derivate der Formel (I) können nach an sich bekannten Methoden hergestellt werden, insbesondere durch die Reaktion des 1-(2-Hydroxyäthyl)-4-hydroxy-2,2,6,6-tetramethylpiperidins der Formel (II)

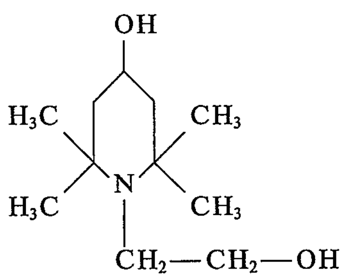

(II)

mit einem reaktionsfähigen Derivat einer Carbonsäure (Säurehalogenid, -anhydrid oder -niederakylester) der Formel (III)

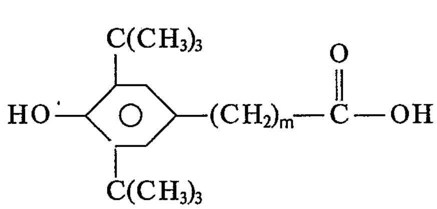

(III)

In Formel (III) hat m die oben angegebene Bedeutung.

Ist das reaktionsfähige Carbonsäure-Derivat ein Ester, so wird die Reaktion vorzugsweise in Gegenwart einer starken Base und in einem inerten organischen Lösungsmittel durchgeführt.

Beispiele solcher Lösungsmitteln sind aromatische und aliphatische Kohlenwasserstoffe, wie Benzol, Toluol, Xylol, n-Heptan, n-Octan oder Iso-octan. Starke Basen sind z. B. Alkalimetall-Verbindungen, wie Natrium-methoxid oder -äthoxid, Kaliumhydroxid oder Lithiumamid; ferner Titansäurederivate, wie Tetraisopropyl-titanat oder Tetrabutyl-titanat. Vorzugsweise wird die Reaktion bei erhöhter Temperatur durchgeführt, z. B. bei 80 – 180° C.

Wird ein Säurehalogenid verwendet, so wird die Reaktion vorzugsweise in Gegenwart eines säurebindenden Mittels und in einem inerten organischen Lösungsmittel durchgeführt. Beispiele solcher Lösungsmittel sind aromatische Kohlenwasserstoffe, wie Benzol, Toluol oder Xylol; halogenierte aliphatische Kohlenwasserstoffe, wie Chloroform oder Trichloräthan; oder Äther, wie Diäthyläther, Tetrahydrofuran oder Dioxan. Säurebindende Mittel sind z. B. Alkalimetallhydroxide, wie NaOH oder KOH, Alkalimetallcarbonate wie $Na_2CO_3$, $K_2CO_3$; oder organische Basen, wie Triäthylamin oder Pyridin. Die Reaktion wird üblicherweise bei Temperaturen zwischen 0° und 130° C durchgeführt.

Wird ein Säureanhydrid verwendet, so kann mit oder ohne Lösungsmittel gearbeitet werden. Wird die Reaktion ohne Lösungsmittel durchgeführt, so wird in der Regel ein Überschuß an Anhydrid eingesetzt. Beispiele von Lösungsmitteln sind aromatische Kohlenwasserstoffe, wie Benzol, Toluol oder Xylol, Äther, wie Dioxan, Tetrahydrofuran oder Diäthylenglycol-dimethyläther. Die Reaktionstemperatur kann bis zu etwa 160° C betragen.

Die erfindungsgemäßen 2,2,6,6-Tetramethylpiperidin-Derivate eignen sich für die Stabilisierung von zahlreichen Polymeren. Beispiele solcher Polymere sind:

Olefin- und Dienpolymere

Zum Beispiel Homopolymere von Olefinen und Dienen (beispielsweise Polyäthylen hoher und niedriger Dichte und quervernetztes Polyäthylen, Polypropylen, Polyisobutylen, Polymethylbuten-1, Polymethylpenten-1, Polyisopren und Polybutadien), Mischungen von solchen Homopolymeren (z. B. Mischungen aus Polypropylen und Polyäthylen, Polypropylen und Polybuten-1 oder Polypropylen und Polyisobutylen) und Copolymere von Olefinen und Dienen (z. B. Äthylen/Propylen-Copolymere, Propylen/Buten-1-Copolymere, Propylen/Isobutylen-Copolymere, Äthylen/Buten-1-Copolymere und Terpolymere von Äthylen und Propylen mit Dienen, wie Hexadien, Dicyclopentadien oder Äthylidennorbornen).

Styrolpolymere

Zum Beispiel Polystyrol, Copolymere von Styrol und $\alpha$-Methylstyrol (z. B. Styrol/Butadien-Copolymere, Styrol/Acrylnitril-Copolymere, Styrol/Acrylnitril/Methylmethacrylat-Copolymere, Styrol/Acrylnitril/Acrylester-Copolymere, Styrol/Acrylnitril-Copolymere, modifiziert mit Acrylesterpolymeren zur Verleihung von Schlagfestigkeit, und Styrolpolymere, modifiziert mit Äthylen/Propylen/Dien-Elastomeren zur Verleihung von Schlagfestigkeit) und Pfropf-Copolymere von Styrol (beispielsweise Polymere, in denen Styrol auf Polybutadien aufgepfropft ist, und Polymere, in denen Styrol und Acrylnitril auf Polybutadien aufgepropft sind, und Mischungen davon mit den vorstehenden Styrol-Copolymeren, die allgemein als Acrylnitril/Butadien/Styrol- oder ABS-Kunststoffe bekannt sind).

Halogenierte Vinyl- und Vinylidenpolymere

Zum Beispiel Polyvinylchlorid, Polyvinylidenchlorid, Polyvinylfluorid, Polychloropren, chlorierte Kautschuke, Vinylchlorid/Vinylidenchlorid-Copolymere, Vinylchlorid/Vinylacetat-Copolymere und Vinylidenchlorid/Vinylacetat-Copolymere.

Von $\alpha,\beta$-ungesättigten Säuren abgeleitete Polymere

und deren Derivate, z. B. Polyacrylate und Polymethacrylate, Polyacrylamide und Polyacrylnitril.

Polymere, abgeleitet von ungesättigten Alkoholen und Aminen

und von deren Acylderivaten oder Acetalen, z. B. Polyvinylalkohol, Polyvinylacetat, Polyvinylstearat, Polyvinylbenzoat, Polyvinylmaleat, Polyvinylbutyral, Polyallylphthalat und Polyallylmelamin, und Copolymere davon mit anderen äthylenisch ungesättigten Monomeren (z. B. Äthylen/Vinylacetat-Copolymere).

Epoxypolymere

Zum Beispiel Homopolymere und Copolymere, abgeleitet von Epoxyden (z. B. Polyäthylenoxyd) und Polymere, abgeleitet von Bis-glycidyläthern.

Polyacetale, Polyalkylenoxyde und Polyphenylenoxyde

Zum Beispiel Polyoxymethylen, Oxymethylen/Äthylenoxyd-Copolymere, Polyoxyäthylen, Polypropylenoxyd, Polyisobutylenoxyd und Polyphenylenoxyde.

Polyurethane und Polyharnstoffe

Polycarbonate

Polysulfone

Polyamide und Copolyamide,
abgeleitet von Diaminen und Dicarbonsäuren und/oder von Aminocarbonsäuren oder den entsprechenden Lactamen, einschließlich Nylon-6, Nylon-6,6, Nylon-6,10, Nylon-11 und Nylon-12.

Polyester,
abgeleitet von Dicarbonsäuren und Dialkoholen und/oder von Hydroxycarbonsäuren und den entsprechenden Lactonen, beispielsweise Polyäthylenglokolterephthalat und Poly-1,4-dimethylol-cyclohexanterephthalat.

Vernetzte Polymere,
abgeleitet von Aldehyden, zusammen mit Phenolen, Harnstoffen oder Melaminen, z. B. Phenol/Formaldehyd-, Harnstoff/Formaldehyd- und Melamin/Formaldehyd-Harze.

Alkydharze
Zum Beispiel Glycerin/Phthalsäure-Harze und Mischungen davon mit Melamin/Formaldehyd-Harzen.

Ungesättigte Polyesterharze,
abgeleitet von Copolyestern von gesättigten und ungesättigten Dicarbonsäuren, mit mehrwertigen Alkoholen sowie von Vinylverbindungen als Vernetzungsmittel und auch halogenierte flammwidrige Modifikationen davon.

Die benötigte Menge der erfindungsgemäßen Stabilisatoren zur wirksamen Stabilisierung von organischen Polymeren hängt von vielen Faktoren ab, wie die Art und Eigenschaften des betreffenden Polymeren, seine beabsichtigte Verwendung und die Gegenwart von anderen Stabilisatoren. Im allgemeinen ist es zufriedenstellend, 0,01 bis 5 Gewichts-% des erfindungsgemäßen Stabilisators, bezogen auf das Gewicht des Polymeren, zu verwenden, jedoch variiert der wirksamste Bereich mit der Art des Polymeren und beträgt beispielsweise 0,01 bis 2,0 vorzugsweise 0,02 bis 1,0 Gewichts-% für Olefin-, Dien- und Styrol-Polymere; 0,01 bis 1,0, vorzugsweise 0,02 bis 0,5 Gewichts-% für Vinyl- und Vinyliden-Polymere und 0,01 bis 5,0, vorzugsweise 0,02 bis 2,0 Gewichts-% für Polyurethane und Polyamide. Gewünschtenfalls können zwei oder mehrere erfindungsgemäße Stabilisatoren gleichzeitig verwendet werden.

Die erfindungsgemäßen Stabilisatoren können leicht in die organischen Polymeren nach üblichen Techniken in jeder geeigneten Stufe vor der Herstellung der entsprechenden Formkörper eingearbeitet werden. Beispielsweise kann der Stabilisator mit dem Polymeren in Form eines trockenen Pulvers vermischt werden, oder eine Suspension oder Emulsion des Stabilisators kann mit einer Lösung, Suspension oder Emulsion des Polymeren vermischt werden.

Die erfindungsgemäßen stabilisierten polymeren Zusammensetzungen können gegebenenfalls auch ein oder mehrere verschiedene übliche Additive enthalten; wie sie z. B. in GB-A-1 401 924, S. 11 – 13, beschrieben sind.

Die Erfindung wird in den folgenden Beispielen näher erläutert. Teile bedeuten darin jeweils Gewichtsteile.

## Beispiel 1

1-[2-{3-(3,5-Di-tert.butyl-4-hydroxyphenyl)propionyloxy}äthyl]-4-{3-(3,5-di-tert.butyl-4-hydroxyphenyl)propionyloxy}-2,2,6,6-tetramethylpiperidin (Stabilisator No. 1)

Ein Gemisch von 2 g 1-(2-Hydroxyäthyl)-4-hydroxy-2,2,6,6-tetramethylpiperidin, 6,4 g Methyl-3-(3,5-di-tert.butyl-4-hydroxyphenyl)-propionat und 0,4 g Lithiumamid wurden während 8 Stunden in 300 ml Toluol bei Rückflußtemperatur gehalten, und gleichzeitig fortlaufend Toluol/Methanol azeotrop abdestilliert. Dabei wurde das Volumen der Reaktion durch laufende Zugabe von Toluol auf 300 ml gehalten. Nach Beendigung der Reaktion wurde das Reaktionsgemisch in Eiswasser gegossen, die Toluolphase abgetrennt und über $MgSO_4$ getrocknet. Das Toluol wurde im Vakuum abdestilliert, und der Rückstand durch Chromatographie in einer Silikagelkolonne gereinigt. Schlußendlich wurde das Produkt aus n-Hexan umkristallisiert. Die weißen Kristalle haben einen Schmelzpunkt von 137 – 138,5° C.

## Beispiel 2

### Hitzestabilitätstest

100 Teile unstabilisiertes Polypropylenpulver (MFI = 15) und 0,25 Teile des in der untenstehenden Tabelle aufgeführten Stabilisators No. 1 wurden vermischt und in einem Brabender Plastographen während 10 Min. bei 200° C homogenisiert. In einer Presse wurden darauf Platten von einer Dicke von 2 – 3 mm gepreßt. In einer Kompressionpreßmaschine wurden bei einer Preßzeit von 6 Min. bei 260° C Folien von 0,5 mm Dicke gepreßt, welche sofort in kaltem Wasser abgekühlt wurden. Aus diesen Folien wurden Prüflinge von 1 × 10 cm gestanzt. Die Prüflinge wurden in einem Umluftofen bei 150° C gebracht und alle 20 Stunden auf Versprödung geprüft. Vergleiche mit bekannten Stabilisatoren wurden auf gleiche Weise durchgeführt, nämlich mit [3-(3,5-Di-tert.butyl-4-hydroxyphenyl)propionyl-oxy]-2,2,6,6-tetramethylpiperidin (A) und mit 4-[3-(3,5-Di-tert.butyl-4-hydroxyphenyl)propionyloxy]-1,2,2,6,6-pentamethylpiperidin (B). Die Resultate sind in der untenstehenden Tabelle I zusammengestellt.

Tabelle I

| Stabilisator No. | Zeit bis zur Versprödung |
|---|---|
| 1 | 500 Stunden |
| A | 80 Stunden |
| B | 100 Stunden |

## Beispiel 3

### Lichtstabilitätstest

Die gemäß Beispiel 2 erhaltenen 0,5 mm dicken Prüflinge wurden in einer hydraulischen Presse während 6 Min. bei 260° C zu 0,1 mm dicken Filmen verpreßt. Aus dem so erhaltenen Film wurden Prüflinge von 50 × 120 mm ausgestanzt, welche in einem Sunshine Weather Meter einer Black Panel Temperatur von 63 ± 3° C ausgesetzt wurden und periodisch auf der Reißfestigkeit geprüft wurden. Die Resultate werden als Verhältnis der Zeit bis die Prüflinge 50% Reißfestigkeit erreichen zur Zeit, wenn unstabilisierte Prüflinge verwendet werden.

Tabelle II

| Stabilisator No. | Verhältnis |
|---|---|
| 1 | 4.5 |

## Beispiel 4

### Verarbeitungsstabilisierung

100 Teile unstabilisiertes Polypropylenpulver und 0,1 Teil der Stabilisatoren der Tabelle III wurden in einem Brabender Plastograph bei 200° C während 10 Min. bei 30 Upm homogenisiert. Die heiße Mischung wurde zu 1 – 2 mm dicken Platten verrollt, aus welcher die Prüflinge geschnitten wurden. Der Schmelzindex (MFI) der Prüfliche wurde gemäß Vorschriften L und B der Test ASTMD-1238-73 gemessen.

Als Vergleich wurden die im Beispiel 2 beschriebenen bekannten Stabilisatoren (A) und (B) eingesetzt.

Tabelle III

| Stabilisator No. | MFI |
|---|---|
| 1 | 5.8 |
| A | 12.9 |
| B | 29.9 |
| ohne | 164 |

Beispiel 5

6 bis 8 mg des Stabilisator No. 1 der Tabelle IV wurden in einer Thermowaage mit 5° C/Min. bei einem Luftstrom von 50 ml/Min. erhitzt. Die Menge des unzersetzten Stabilisators wurden bei 250—300° C gemessen.

Tabelle IV

| Stabilisator No. | Noch vorhandener Stabilisator (%) | |
|---|---|---|
| | 250°C | 300°C |
| 1 | 100.0 | 95.8 |

**Patentansprüche**

1. 2,2,6,6-Tetramethylpiperidin-Derivate der Formel I

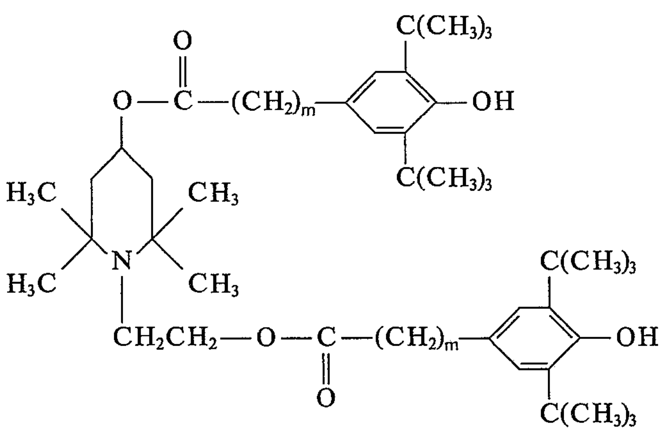

(I)

worin m 0, 1 oder 2 bedeutet.

2. Verbindungen gemäß Anspruch 1 der Formel I, worin m 2 ist.

3. Stabilisiertes synthetisches Polymer, enthaltend ein 2,2,6,6-Tetramethylpiperidin-Derivat der Formel I nach Anspruch 1.

4. Polymer gemäß Anspruch 3, enthaltend die Verbindung nach Anspruch 2.

5. Verfahren zum Herstellen von Verbindungen der Formel I gemäß Anspruch 1, dadurch gekennzeichnet, daß man 1-(2-Hydroxyäthyl)-4-hydroxy-2,2,6,6-tetramethylpiperidin der Formel II

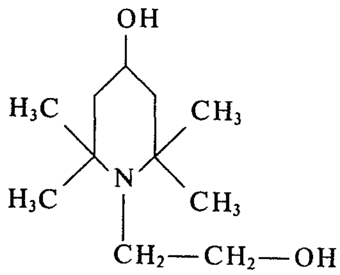

(II)

mit einem reaktionsfähigen Derivat einer Carbonsäure der Formel III

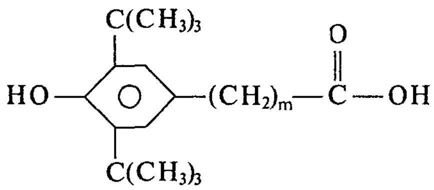

(III)

umsetzt, in der m die im Anspruch 1 angegebene Bedeutung hat.

## Claims

1. A 2,2,6,6-tetramethylpiperidine derivative of the formula I

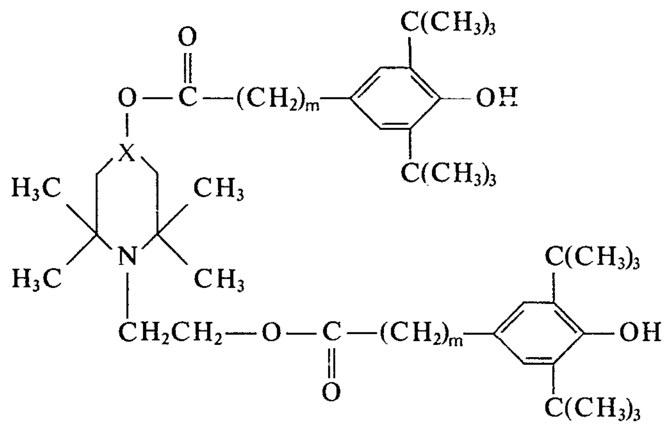

(I)

wherein
m is 0, 1 or 2.

2. A compound according to Claim 1 of the formula I wherein m is 2.

3. A stabilised synthetic polymer containing a 2,2,6,6-tetramethylpiperidine derivative of the formula I according to Claim 1.

4. A polymer according to Claim 3, which contains a compound according to Claim 2.

5. A process for producing a compound of the formula I according to Claim 1, characterised in that 1-(2-hydroxyethyl)-4-hydroxy-2,2,6,6-tetramethylpiperidine of the formula II

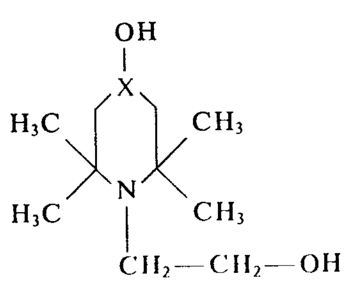

(II)

is reacted with a reactive derivative of a carboxylic acid of the formula III

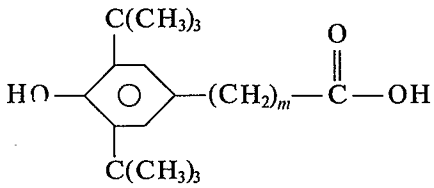

(III)

in which m has the meaning defined in Claim 1.

**Revendications**

1. Dérivés de la tétramethyl-2,2,6,6-pipéridine qui répondent à la formule I

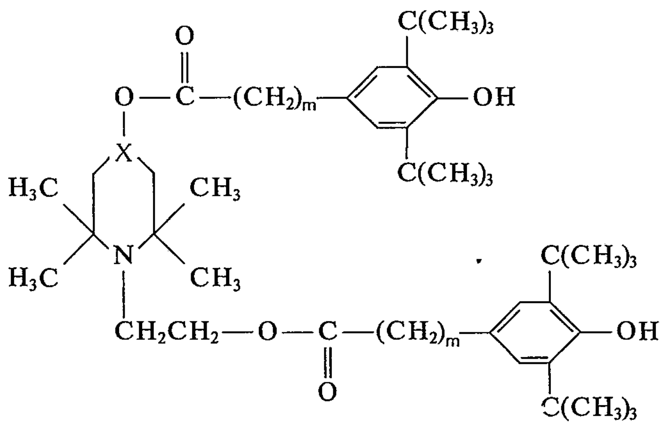

(I)

dans laquelle m est égal à zéro, à 1 ou à 2.

2. Composés de formule I selon la revendication 1 dans lesquels m est égal à 2.

3. Polymère synthétique stabilisé qui contient un dérivé de la tétraméthyl-2,2,6,6 pipéridine de formule I selon la revendication 1.

4. Polymère selon la revendication 3, qui contient le composé de la revendication 2.

5. Procédé de préparation de composés de formule I selon la revendication 1, procédé caractérisé en ce qu'on fait réagir 1-(hydroxy-2 éthyl)-1 hydroxy-4 tétraméthyl-2,2,6,6 pipéridine, corps qui répond à la formule II

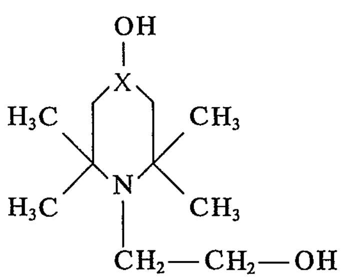

(II)

avec un dérivé réactif d'un acide carboxylique réspondant à la formule III

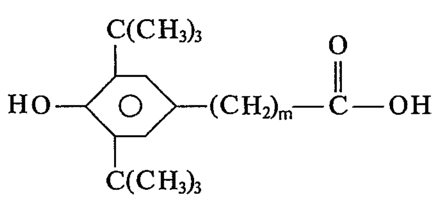

(III)

dans laquelle m a la signification donnée à la revendication 1.